# EUROPEAN PATENT SPECIFICATION

(11) **EP 4 161 622 B1**
(45) Date of publication and mention of the grant of the patent: **07.08.2024**
(21) Application number: 21737151.7
(22) Date of filing: 09.06.2021
(51) Int. Cl.: A61M 16/10, A61M 16/00, A61M 16/12, A61M 16/20

(54) **VENTILATOR**
VENTILATOR
VENTILATEUR

(30) Priority: 09.06.2020 PT 2020116488
(43) Date of publication of application: 12.04.2023
(73) Proprietor: CEIIA - Centro de Engenharia e Desenvolvimento (Associação), 4450-017 Matosinhos (PT)
(72) Inventor: COVAS OLIVEIRA, Hélder Filipe, 4450-017 Matosinhos (PT); SIMAS SILVA, Helena Luísa, 4450-017 Matosinhos (PT); LOPES FERREIRA LUÍS, João Pedro, 4450-017 Matosinhos (PT); CORREIA DE OLIVEIRA E SILVA, José Roque, 4450-017 Matosinhos (PT); DE ENCARNAÇÃO PALMA FELIZARDO, José Rui, 4450-017 Matosinhos (PT); FERREIRA MENDES BRAGA, Luis Miguel, 4450-017 Matosinhos (PT); CASAL MARTINS DE OLIVEIRA, Manuel Pedro, 4450-017 Matosinhos (PT); DA SILVA MAGALHÃES, Raquel, 4450-017 Matosinhos (PT); DE AZEVEDO MAGALHÃES, Rui Miguel, 4450-017 Matosinhos (PT); RAMOS REBELO, Tiago Alexandre, 4450-017 Matosinhos (PT)
(74) Representative: Couto, Cláudia
(86) International application number: PCT/IB2021/055054
(87) International publication number: WO 2021/250582

(56) References cited:
- EP-A2- 0 860 175
- US-A- 5 237 987
- US-B1- 9 180 266

## Description

### Technical Domain

The present invention describes a pulmonary ventilator for continuous mechanical ventilation support to patients with acute pneumonia.

### Background

Coronavirus 2019 disease (COVID-19) is the result of an infection caused by coronavirus-2, related to severe acute respiratory syndrome (SARS-CoV-2), which leads infected patients to express symptoms that can range from mild respiratory tract symptoms to severe respiratory distress and sepsis, which can be lethal. In the fight against the COVID-19 pandemic, the race for mechanical ventilation equipment has intensified at a global level. It is known that the lack of ventilators is currently one of the greatest difficulties because it limits the clinical response to severe and potentially fatal respiratory problems. According to WHO, 14% of those infected with COVID-19 develop pneumonia and 5% progress to a very critical condition, evolving into bilateral interstitial pneumonia, which attacks both lungs and causes the patient to need invasive ventilation to be able to breathe and fight the disease.

Currently, all over the world, the fight against COVID-19 is growing every day. The devastating consequences of this highly contagious infectious disease instigate serious anguish in most national health care systems, which are increasingly affected by the lack of skilled health professionals and adequate medical equipment to treat all affected patients.

In an attempt to provide support and assistance to infected patients, most countries worldwide have unleashed an unimaginable search for ventilation equipment that could help significantly reduce the mortality rate of patients. This unexpected demand has caused almost all countries in the world with a shortage of adequate medical ventilation equipment to compete in the market to acquire this equipment. Unfortunately, most countries, for a variety of reasons, will not be able to acquire or provide this support to all patients in need thereof. The supply of such equipment by known manufacturers is insufficient to meet the demand and, therefore, the search for viable alternatives is necessary.

US5931159 describes a pulmonary ventilator that is quick to manufacture for delivering a mixture of oxygen and air to a patient as an inspiration gas. The pulmonary ventilator includes a compressor with an inlet line to absorb oxygen and gas and deliver a mixture of oxygen and gas to a main flow line as inspiration gas to the patient. A recycle line is connected to the main flow line and the compressor inlet line and a control arrangement is provided to allow a portion of the inspiration gas to flow into the main flow line in excess of the inspiration gas used by the patient on the recycling line to supply the compressor inlet line.

US9180266B1 discloses a ventilator that enables the operator to enter into the microprocessor estimate of a patient's individual characteristic, such as weight, which the microprocessor uses to control delivered tidal volume and other parameters to match the patient. The operator can select one of several ventilator operational modes (intube, mask, CPR) . Sensors input data to the microprocessor to maintain parameter optimizations and accuracy. Visual/audible alarms and tools activate when one or more parameters exceed or fail to exceed predetermined values for patient's weight. Manual over-ride is available. The ventilator has a quick start capability in which the operator turns on power, selects the automatic operating mode, enters patient's characteristic, selects control option starting automatic ventilation of proper volumes inhalation/exhalation periods, pressure, and oxy-air mixture.

EP0860175A2 discloses a ventilator for intensified breathing, whereby a restrictor valve for an over-pressure in the system, a valve allowing breathing from the atmosphere and a directional valve for inhalation are combined to provide a single safety valve (21) arranged in a gas mixture and inhalation conduit assembly (7, 8), said safety valve being arranged to be controlled by an electric control system (26) of the ventilator. The invention also relates to a valve of the above type.

### Summary

The present application describes a pulmonary ventilator for use in patients, composed of a control module (112); a compressed air inlet (101); a filter pressure regulator (103), fed by the compressed air inlet (101); an air solenoid valve (107), fed by the filter pressure regulator (103), controlled by the control module (112); an air flow sensor (109), fed by the air solenoid valve (107) and read by the control module (112); an O₂ inlet (102); an O₂ tank (104), fed by the O₂ inlet (102); an O₂ solenoid valve (108), fed by the O₂ tank (104), controlled by the control module (112); an O₂ flow sensor (110), fed by the O₂ solenoid valve (108) and read by the control module (112); a gas mixture line (111), fed by the air flow sensor (109) and the O₂ flow sensor (110); wherein the gas mixture line outlet (111) ensures the supply of the gas mixture to the patient (121).

The pulmonary ventilator additionally comprises an air pressure sensor and an O₂ pressure sensor, read by the control module, in order to ensure the stabilization of the air inlet pressure level and the O₂ inlet pressure level.

In a proposed embodiment, the pulmonary ventilator additionally comprises an Ar+O₂ pressure sensor, an O₂ sensor, an emergency breathing valve and an overpressure valve.

In another proposed embodiment, the pulmonary ventilator further comprises a flow sensor, an expiration pressure sensor, an expiratory solenoid valve and an air outlet.

In another proposed embodiment, the control module reads data from the Ar+O₂ pressure sensor, the O₂ sensor, the flow sensor, the expiratory pressure sensor and controls the expiratory solenoid valve, in order to ensure the quality standards for supplying the mixture of gases to the patient. In another proposed embodiment, the emergency breathing valve ensures the supply of air to the patient in case of internal failure.

In another proposed embodiment, the overpressure valve allows for discharges of the mixture when the pressure exceeds the desired limit.

In another proposed embodiment, the control module comprises a volume control mode (CMV), a pressure control mode (PCV), a pressure-regulated volume control mode (PRVC) and a pressure support mode (PSV).

In another proposed embodiment, the control module adjusts the volume of the gas mixture delivered to the patient in a range from 250 to 800 mL per respiratory cycle.

In another proposed embodiment, the control module adjusts the oxygen concentration in the gas mixture ensuring supplying with values between 21% and 100%.

In another proposed embodiment, the control module regulates the number of inspirations per minute, allowing adjustments between 5 and 50 cycles per minute.

In another proposed embodiment, the pulmonary ventilator further comprises a communication module to enable remote network integration, control and monitoring of patients.

### Brief description

In this pandemic context and given the growing need for clinical devices for invasive and/or non-invasive ventilation, a medical ventilator that is quick to manufacture has been developed, which is innovative and capable of supporting patients with acute respiratory failure, such as that associated with COVID-19, all of this being carried out in a short period of time, allowing its modular evolution into new versions with the greatest possible technical and evolutionary incorporation.

The present invention relates to a pulmonary ventilator that is quick to manufacture. In the initial phase of the developed product, the ventilator started from a conceptual basis based on the use of common components on the market. Although the electrical and mechanical elements are of a commercial nature, the functional and operational concept and model for the control and management of the equipment is fully developed in order to ensure the best performance and safety for the user of the equipment.

The ventilator provides continuous mandatory ventilation support to patients in the early stages of acute pneumonia treatment, wherein pressure-regulated volume control ventilation mode (PRVC), as well as pressure-controlled ventilation (PCV) and volume-controlled ventilation (CMV) are required. In addition, the developed ventilator features a spontaneous respiratory pressure support mode (triggered assisted ventilation) for patients with limited autonomous breathing capacity, in the recovery phase (PSV mode).

In addition, the new equipment makes it possible to control all essential parameters that must be regulated by physicians when caring of acute respiratory patients; monitor, at all times, critical aspects of the patient and issue alarms whenever there is a malfunction or an unexpected parameter; be able to integrate with the existing hospital gas infrastructure and be compatible with the invasive consumables approved for medical use (respiratory systems, filters, endotracheal tubes, etc.) currently in use in hospitals.

The objective of this system is to improve and consider, in evolutionary terms, that the standards initially considered ensure the incorporation of new elements, based on the need to develop new features of connectivity and remote monitoring compared to the initial version, making it also more compact and with greater mobility, with integration availability by incorporating additional modular components and with a practical and simple-to-operate reference design. The optimization and improvement of the architecture for the developed ventilator, both in terms of software and hardware, took into account ensuring and providing the critical subsystems and modules of the ventilation function with fault tolerance mechanisms implemented in hardware and software, through the creation of redundant circuits and routines. This application intends to be technologically disruptive with the equipment currently on the market, in conceptual and technological terms. Therefore, and considering the innovation chain, the proposed ventilator considers the integration of remote connectivity through the incorporation of a communication and assisted control module. This functionality will allow the integration of several ventilators as networks of objects that can be managed remotely and that allow the treatment of patients between hospital units. This remote monitoring and control of the ventilators from a network connection point of view, through the additional integration of an intelligence module, enables remote management in an aggregating and concentrated way, minimizing local monitoring failures. Furthermore, and as the number of specialists able to work with this equipment is often reduced, the incorporation of the possibility of remote operation/monitoring of ventilators simultaneously allows reducing the risk and duration of exposure/contamination of health professionals, as well as providing this treatment, and the associated medical decisions, to patients dispersed in different hospital units. With this feature, the treatment of patients becomes possible outside hospital units.

Digital security, being also one of the key issues on the agenda, is also ensured, allowing for remote operations and access to data, including issues associated with cybersecurity and possible maintenance or software upgrade.

The remote control module enhances the development of an information system, as well as remote training through assisted control and suggestion of ideal configuration parameters, depending on the patient's profile, based on historical data.

Another concept also introduced in the ventilator involves the development and use of an internal architecture of subsystems and modules, both in terms of software and hardware, which are distributed and fault-tolerant. The integration and use of security mechanisms is ensured by a communication and command design adapted to the need, and in hardware there is a guarantee of support in the integration between the application and the management and control software.

Given the application of the modularity concept, a product with an optimized and improved architecture naturally emerges that allows optimizing production processes with quick assemblies, using components and materials existing and/or produced by local industry, and therefore, based on chain proximity supply or with local stock.

The characteristics identified above lead to the development of a new industrialization model based on network integration of several production units managed from a localized production cell, with centralized production, order and purchase management.

As key requirements for the development of the ventilator, it has been ensured that the equipment is easy and intuitive to use by healthcare professionals. The equipment is safe and reliable, not jeopardizing the health status of patients, nor harming them, allowing for simplified cleaning and decontamination conditions associated with the use thereof.

The ventilator ensures continuous and fault-free operation for a minimum period of 15 days, 24 hours a day, and if necessary, some components may be replaced at the end of this cycle. A great asset of the concept behind the development of this ventilator is the fact that the equipment is produced from common materials and components, available through local supply chains, as well as from manufacturing technologies available in national territory, without complex integrations or excessive delivery times. It is technically ensured that the equipment is compatible with the standard air and oxygen infrastructure installed in hospitals, but it also allows the integration of air compression systems or portable oxygen bottles or concentrators, as long as the minimum flow and pressure are ensured.

The ventilator allows high mobility and can be used in hospital context, including field hospitals, and is compatible with standard invasive medical components, including tubing, fittings, mouthpieces, etc. The ventilator's windpipes are also compatible with HME, HMEF and HEPA humidifying and cleaning filters, or equivalent, allowing for easy installation thereof.

In terms of technical requirements, the ventilator produced ensures the supply of an adjustable gas mixture volume in a range from 250 - 800 mL per respiratory cycle, being able to provide an air flow of up to 100L/min in periods of less than 25 milliseconds. The ventilator also allows the maintenance of a positive end-expiratory pressure (PEEP), on average between 0 and 40 cmH₂0, providing an oxygen concentration in the gas mixture equal to or greater than 21% up to 100%, allowing an unconscious patient, unable to perform active breathing autonomously, to breathe.

The number of inspirations per minute is internally controlled, allowing it to vary between 5 and 50 cycles. With regard to the electrical supply of the equipment, this is ensured by an external source, during its normal operation, with the system additionally having a support battery that ensures the operation thereof for a maximum period of 5 hours without the need to connect to the energy network. The equipment further includes a configurable digital control system using analog buttons and a touch screen that may include diversified information, including pressure, flow and volume graphs. The control of parameters associated with the volume control mode (CMV) is ensured, as well as the control of parameters associated with the pressure control mode (PCV) and the pressure-regulated volume control mode (PRVC) and parameters associated with the pressure support mode (PSV).

The ventilator further ensures at least the monitoring of the following parameters, ensuring the issuing of alarms if they deviate from the preset ranges:
- Disconnection/sudden pressure drop;
- Respiratory rate (RR) - maximum and minimum or without respiratory movements - in apnea;
- Minute Volume - minimum and maximum alarm;
- Pressure - maximum defined by the user;
- Loss of AC power;
- Failure to supply O₂ and/or Air;
- FiO₂ - Fraction of inspired oxygen deviated from the set value;
- Inspiratory pressure - deviation from the default value;
- PEEP - deviation from the default value.

The ventilator further monitors the following parameters:
- Pressure vs time;
- Flow vs time;
- Volume vs time;
- Patient's respiratory rate;
- Patient's effective I:E ratio;
- Inspiratory time;
- Expiratory time;
- Compliance;
- Resistance;
- Oxygen supply pressure;
- Air supply pressure;
- Pressure plateau;
- Peak pressure;
- Average pressure;
- Positive end-expiratory pressure - PEEP;
- Inspired/expired tidal volume;
- Inspired/expired minute volume;
- Fraction of inspired oxygen - FiO₂;
- Ventilation time.

### Brief Description of The Figures

For an easier understanding of the present application, figures are herein attached, which represent embodiments which however are not intended to limit the art herein disclosed.

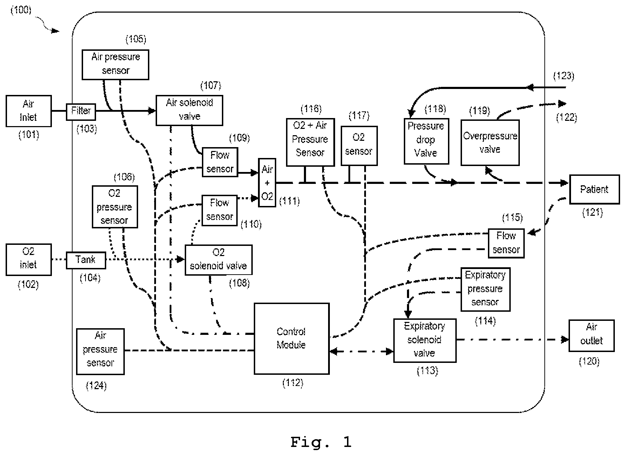

Figure 1 illustrates the operating scheme and respective components of the ventilator (100), wherein:
- (101) -: Compressed air inlet;
- (102) -: O₂ inlet;
- (103) -: Filter pressure regulator;
- (104) -: O₂ tank;
- (105) -: Air pressure sensor;
- (106) -: Oxygen pressure sensor;
- (107) -: Air solenoid valve;
- (108) -: Oxygen solenoid valve;
- (109) -: Air flow sensor;
- (110) -: O₂ flow sensor;
- (111) -: Gas mixture line (inspiration);
- (112) -: Control module;
- (113) -: Expiratory solenoid valve;
- (114) -: Expiratory pressure sensor;
- (115) -: Expiratory flow sensor;
- (116) -: Inspiratory mixture pressure sensor;
- (117) -: Oxygen concentration sensor;
- (118) -: Emergency breathing valve;
- (119) -: Overpressure valve;
- (120) -: Inspiratory gas mixture outlet;
- (121) -: Patient;
- (122) -: Overpressure valve outlet;
- (123) -: Room air inlet for the emergency breathing valve;
- (124) -: Room pressure sensor.

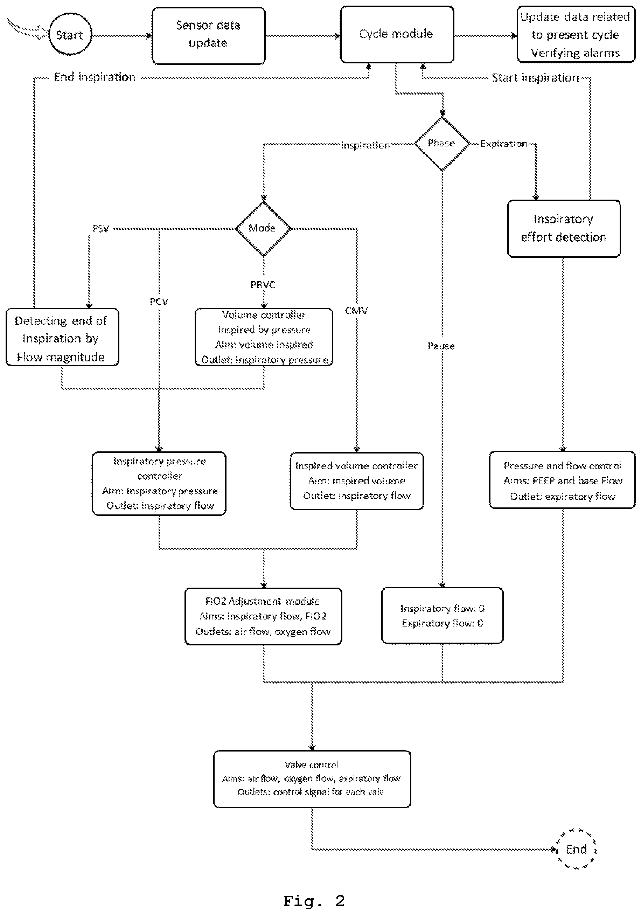

Figure 2 illustrates the flowchart of the control algorithm (200) of the ventilator (100) wherein:
- (201) -: Entry into the control cycle;
- (202) -: Update of sensor readings;
- (203) -: Cycle module;
- (204) -: Leak/Inspiratory detection;
- (205) -: Pi analysis;
- (206) -: Overpressure/start expiration alarm;
- (207) -: Phase cycle analysis;
- (208) -: Operating mode: PCV, CMV or adaptive PCV;
- (209) -: PCV/Pi=Ps mode setpoint;
- (210) -: Adaptive PCV/Pi mode setpoint;
- (211) -: CMV/Fi = Vs/ti mode setpoint;
- (212) -: FiO₂ mixture and outflow Ar-V, OXY-V;
- (213) -: Inspiratory and expiratory shut-off valves;
- (214) -: Control/Pe=PEEP step setpoint;
- (215) -: Loop;
- RR -: Respiratory rate;
- t -: Total time since the beginning of the cycle;
- ti -: Inspiratory time;
- te -: Expiratory time;
- tip -: Inspiratory pause time;
- Pi -: Inspiratory circuit pressure;
- Pe -: Expiratory circuit pressure;
- Ps -: Pressure setpoint;
- Ptr -: Pressure trigger;
- Fi -: Inspiratory circuit flow;
- Fe -: Expiratory circuit flow;
- Ftr -: Trigger flow;
- FiO₂ -: Fraction of inspired oxygen;
- VT -: Tidal volume;
- VS -: Volume setpoint.

Figure 3 illustrates the control interface (300) of the ventilator (100) via a touchscreen LCD.

Figure 4 illustrates the operating diagram of the software architecture (400) of the ventilator (100) wherein:
- (401) -: Main cycle;
- (402) -: Reading of sensor data;
- (403) -: Resetting sensors and calibration;
- (404) -: Calculating sensor data;
- (405) -: Input data;
- (406) -: Input modules;
- (407) -: Presentation of data;
- (408) -: Alarms;
- (409) -: Actuator valves.

### Description of embodiments

Referring to the figures, some embodiments are now described in more detail, which are not intended, however, to limit the scope of the present application.

The present application describes a pulmonary ventilator (100), which is portable and for use in a hospital environment. It was prepared to work with conventional supply of oxygen (102) and pressurized air, typically found in any hospital infrastructure. In order to broaden its operating spectrum, it was also designed for use in transport or in remote situations, using pressurized O₂ and Air reservoirs. The main power supply is the typical 230V alternating power supply, which will operate for short periods with a 12V rechargeable battery when connected to the main power.

The system developed for the ventilator, in one of the proposed embodiments, consists of a simplified structure in stainless steel mounted on wheels, in which four metal boxes with an IP66 environmental protection index are installed. Two of said boxes are mounted on the lower part of the body, and two are mounted on the upper part of the structure. These boxes form the encapsulation of all pneumatic, electrical and control systems.

In order to simplify the project, and in particular the assembly process, the air and oxygen inlets are placed in one of the lower boxes, and the components related to energy and power supply are integrated in the other lower box. As for the upper boxes, one contains all components related to the inspiratory line, including the controls for the mixture of oxygen and air, and the other includes all components that are part of the expiratory line, as well as the electronic components and the control compartment that is hermetically sealed in a box with IP65 protection index.

The ventilator is generally composed of three subsystems:
- Air/oxygen circuit where gases circulate from the inlet thereof until reaching the patient;
- Electronics and control responsible for the analysis, control and mixture of gases in the air/oxygen circuit;
- Energy that is responsible for powering the entire system.

The compressed air inlet (101) of the ventilator (100) can be connected to hospital compressed air infrastructure, an air compressor or compressed air bottles.

The O₂ inlet (102) of the ventilator (100) can be connected to an oxygen bottle or hospital oxygen infrastructure via an oxygen pressure regulator.

Since the supply of oxygen in the infrastructure is made at a pressure higher than that intended for the operation of the ventilator and, as it suffers some fluctuations in its value, a pressure regulator is used to ensure that the pressure for the patient pneumatic system is within recommended values (approximately 4 bar). Once regulated, the oxygen is stored in a specific medical reservoir that will stabilize the pressure thereof.

For pressurized air, the approach is similar using a pressure regulator with a filter (103). However, the ability to filter the air is added, ensuring that the air reaching the patient is free of impurities and particles.

The control and electronic systems architecture approach considers the use of two solenoid valves (107, 108) in the inspiratory line to control the intake of air and O₂ in the circuit. Using data from the flow sensors (109, 110), air pressure and O₂, we can precisely control the gas mixture line (111) (air and O₂) supplied to the patient (121), also allowing the control of FiO₂ and respiratory rate. Furthermore, the control can be performed in different modes such as volume, pressure and pressure/volume adaptive ventilation.

In the expiratory line, an expiratory flow sensor (115) and a pressure sensor (114) have also been integrated, which will allow to control the expiratory solenoid valve (113). This allows controlling the patient's expiration (121) and implementing PEEP control, one of the main requirements of the ventilator.

Overpressure valve (119) opens if excessive pressure is identified. Emergency breathing valve (118) is opened to supply external air to the patient (121) if the ventilator system fails.

The operator of the ventilator (100) can monitor and control the operation of the system using the interface module, which is directly connected to the control module (112), and incorporates an LCD touchscreen for inputting monitoring reference values.

Before mixing the gases is carried out (112), a pressure control (105, 106) and air flow and oxygen flow control (110) is performed. Two solenoid valves (107, 108) are responsible for allowing the entry of each one of the gases to the next stage, ensuring both by flow and pressure that the mixture is made in the proportions desired by the operator. After mixing, the pressure is again controlled in a pressure sensor of the inspiratory mixture (116) as well as the O₂ concentration also in an oxygen concentration sensor (117) allowing to accurately measure the mixture that will be delivered to the patient. When the expiratory valve (113) opens, the patient enters the expiratory cycle by directing air back to the ventilator. At this stage, the air flow and its pressure are measured and displayed on the display to the user who can adjust the parameters if desired. The sensor data is monitored in real time by the electronic control module (112), allowing to generate alarms and regulate command signals for the solenoid valves depending on user commands.

Pressure control is critical to ensure that the patient is oxygenated as intended by the medical team, thus ensuring his/her safety, not imposing regimes or overpressure or underpressure. Taking this requirement into account, a purely mechanical system was implemented in the patient's inspiratory channel to ensure the safety of the ventilator's operation. On the one hand, the overpressure channel, monitored by the respective valve (119), allows the mixture to be discharged when the pressure exceeds the desired limit for the patient, ensuring that the patient is not exposed to risky pressure. On the other hand, if due to an event such as a significant hole in the pipes or failure of any component that imposes a lower pressure regime than that desired in the inspiratory channel, the underpressure channel, through the emergency breathing valve (118), allows injecting air into the system (at room pressure) to mitigate this effect.

The system is also composed of a power module, to ensure and manage the power supply to all components that make up the ventilator. The energy system includes the incorporation of a battery that will supply support energy in the event of a power failure in the network or if there is a need to move the patient to another location. The ventilator power system is responsible for converting the mains voltage (regardless of the power supply) to the desired voltages inside the ventilator (3.3V, 5V and 12V) through DC-DC voltage regulators. The latter, together with the inclusion of relays and fuses, allow to protect both the equipment and the patient from voltage and current peaks that may occur.

All tubes used in the pneumatic connections of the air/oxygen circuit are Perfluor-alkoxyalkan (PFA) plastic, similar to those typically used in compressed air installations, including ventilators. The use of this type of tubes is compatible with the food industry, and its use is approved by the FDA (US Food and Drug Administration). This material was chosen for its ability to withstand a wide range of pressures and temperatures, namely pressures between -0.95 bar and +15 bar and temperatures between -20° Celsius and +150° Celsius. Since it is easily washable and allows the use of quick connections, its use is an advantage both in the assembly and in the potential repair of the ventilator.

The ventilator's user interface system, based on the use of a touchscreen LCD display, allows
- presenting relevant magnitudes for effective ventilation (PPEAK, PEEP, MVE, TVE, etc.);
- selecting ventilation targets and adjusting parameters;
- switching between ventilator operating modes;
- adjusting windows to visualize desired graphics (with automatically adjustable scale);
- setting alarms for immediate identification of preprogrammed events;
- adjusting display parameters (contrast, brightness, etc.);
- ventilator power mode and respective battery charge status (when operating in this mode);

Figure 2 illustrates the flowchart of the control algorithms (200) of the ventilator (100) that govern system operation.

This algorithm allows controlling ventilation in 3 different modes: volume control CMV (211), pressure control PCV (209) and adaptive pressure control (210). The algorithm uses the input data from the HMI to define the control parameters. The control allows setting the inspiratory pause, I: E ratio, FiO₂ and PEEP setpoints. Control is also dynamic and adapts to variations introduced in the system.

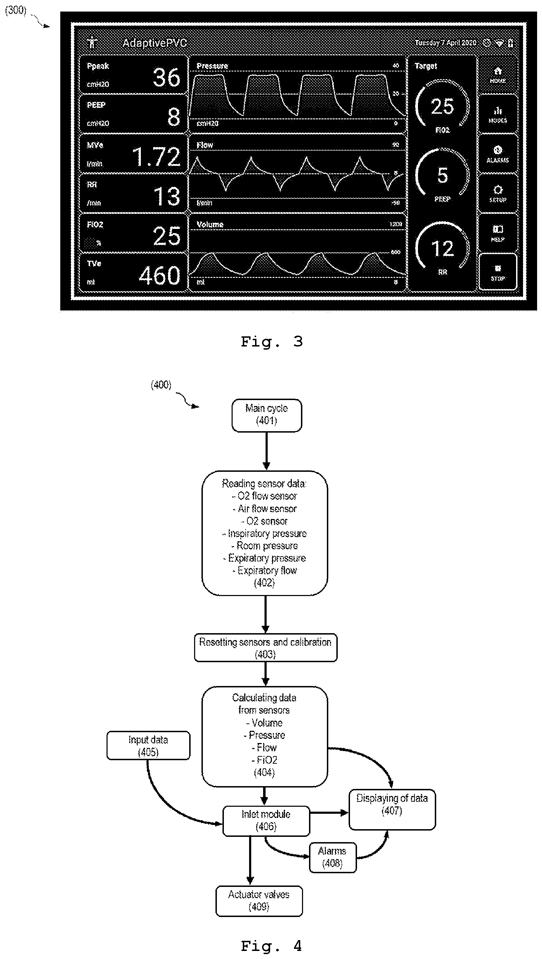

The software architecture (400) shown in Figure 4 was defined to allow control of all variables and is necessary to meet all operating requirements.

The software will run in a continuous cycle, which starts by reading all sensors (402), sets the zero reference to all values and starts a calibration procedure (403). The raw data from the sensors are calculated to pass the correct values to the control routine (404).

On the HMI (406), the user must select the ventilation control mode and enter all necessary parameters (405). The Control Module will adapt the control algorithm according to the selected mode and input parameters. The control module activates the valves (409) and passes the data to be displayed to the HMI screen (407).

An alarm subroutine (408) runs in parallel, reading the system parameters, ensuring that any abnormal value is signaled to the user via the HMI.

The invention is defined in independent claim 1. Preferred embodiments are matter of the appended dependent claims.

## Claims

1. Pulmonary ventilator (100) for use in patients (121), comprising:
- a control module (112);
- a compressed air inlet (101);
- an 02 inlet (102);
- an air pressure sensor (105) and an 02 pressure sensor (106), read by the control module (112), in order to ensure the stabilization of the compressed air inlet pressure level and the 02 inlet pressure level;
- an 02 tank (104), fed by the 02 inlet (102), the 02 tank (104) disposed between the 02 inlet (102) and the oxygen pressure sensor (106), suitable to enable pressurized 02 storage ;
- a filter pressure regulator (103), fed by the compressed air inlet (101), the filter pressure regulator (103) disposed between the compressed air inlet (101) and the air pressure sensor (105), suitable to filter the air thereto provided,
- an air solenoid valve (107), fed by the filter pressure regulator (103), controlled by the control module (112);
- an air flow sensor (109), fed by the air solenoid valve (107) and read by the control module (112);
- an 02 solenoid valve (108), fed by the 02 tank (104), controlled by the control module (112);
- an 02 flow sensor (110), fed by the 02 solenoid valve (108) and read by the control module (112); and
- a gas mixture line (111) for supplying a gas mixture to a patient (121), fed by the air flow sensor (109) and by the 02 flow sensor (110) which are controlled by means of data provided to the control module (112).

2. Pulmonary ventilator (100) according to claim 1, **characterized in that** it further comprises an inspiratory mixture pressure sensor (116), an oxygen concentration sensor (117), an emergency breathing valve (118) and an overpressure valve (119).

3. Pulmonary ventilator (100) according to claim 1, **characterized in that** it further comprises an expiratory flow sensor (115), an expiratory pressure sensor (114), an expiratory solenoid valve (113) and an inspiratory gas mixture outlet (120).

4. Pulmonary ventilator (100) according to claim 1, **characterized in that** the control module (112) reads data from the inspiratory mixture pressure sensor (116), the oxygen concentration sensor (117), the expiratory flow sensor (115), the expiratory pressure sensor (114) and controls the expiratory solenoid valve (113), in order to ensure the quality standards of the gas mixture supply to the patient (121).

5. Pulmonary ventilator (100) according to claims 1 and 2 **characterized in that** the emergency breathing valve (118) ensures the supply of air to the patient (121) in the event of an internal failure.

6. Pulmonary ventilator (100) according to claims 1 and 2 **characterized in that** the overpressure valve (119) allows for discharges of the mixture when the pressure exceeds the desired limit.

7. Pulmonary ventilator (100) according to claim 1, **characterized in that** the control module (112) comprises a volume control mode, a pressure control mode, a pressure-regulated volume control mode, and a pressure support mode.

8. Pulmonary ventilator (100) according to claim 1, **characterized in that** the control module (112) adjusts the volume of the gas mixture delivered to the patient in a range from 250 to 800 mL per respiratory cycle.

9. Pulmonary ventilator (100) according to claim 1, **characterized in that** the control module (112) adjusts the oxygen concentration in the gas mixture ensuring supplying with values between 21% and 100%.

10. Pulmonary ventilator (100) according to claim 1, **characterized in that** the control module (112) regulates the number of inspirations per minute, allowing the adjustment between 5 and 50 cycles per minute.

11. Pulmonary ventilator (100) according to claim 1, **characterized in that** it further comprises a communication module to enable remote network integration, control and monitoring of patients.

## Patentansprüche

1. Lungenbeatmungsgerät (100) zur Anwendung bei Patienten (121), bestehend aus:
- einem Steuermodul (112);
- einem Drucklufteinlass (101);
- einem O₂-Einlass (102);
- einem Luftdrucksensor (105) und einem O₂-Drucksensor (106), die vom Steuermodul (112) gelesen werden, um die Stabilisierung des Druckniveaus des Drucklufteinlasses und des O₂ Eingangsdruckniveaus sicherzustellen;
- einem O₂-Tank (104), der vom O₂-Einlass (102) gespeist wird, wobei der O₂-Tank (104) zwischen dem O₂-Einlass (102) und dem Sauerstoff-Drucksensor (106) angeordnet ist und geeignet ist, die Speicherung des unter Druck stehenden Sauerstoffs zu ermöglichen;
- einem Filterdruckregler (103), der vom Drucklufteinlass (101) gespeist wird, wobei der Filterdruckregler (103) zwischen dem Drucklufteinlass (101) und dem Luftdrucksensor (105) angeordnet ist und zur Filterung der dafür vorgesehenen Luft geeignet ist;
- einem Luft-Magnetventil (107), das vom Filterdruckregler (103) gespeist wird und vom Steuermodul (112) gesteuert wird;
- einem Luftmengenmesser (109), der vom Luft-Magnetventil (107) gespeist und vom Steuermodul (112) gelesen wird;
- Einem O₂-Magnetventil (108), das vom O₂-Tank (104) gespeist und vom Steuermodul (112) gesteuert wird;
- Einem O₂-Durchflusssensor (110), der vom O₂-Magnetventil (108) gespeist und vom Steuermodul (112) gelesen wird, und
- eine Gasgemischleitung (111) zur Versorgung eines Patienten (121) mit einem Gasgemisch, die vom Luftmengenmesser (109) und vom O₂-Durchflusssensor (110) gespeist wird und über die dem Steuermodul (112) bereitgestellten Daten gesteuert wird.

2. Lungenbeatmungsgerät (100) gemäß Anspruch 1, **dadurch gekennzeichnet, dass** es weiterhin einen Drucksensor für die inspiratorische Mischung (116), einen Sauerstoffkonzentrationssensor (117), ein Notfall-Atemventil (118) und ein Überdruckventil (119) umfasst.

3. Lungenbeatmungsgerät (100) gemäß Anspruch 1, **dadurch gekennzeichnet, dass** es ferner einen exspiratorischen Durchflusssensor (115), einen exspiratorischen Drucksensor (114), ein exspiratorisches Magnetventil (113) und einen inspiratorischen Gasgemisch-Auslass (120) aufweist.

4. Lungenbeatmungsgerät (100) gemäß Anspruch 1, **dadurch gekennzeichnet, dass** das Steuermodul (112) die Daten des Drucksensors für die inspiratorische Mischung (116), des Sauerstoffkonzentrationssensors (117), des exspiratorischen Durchflusssensors (115) und des exspiratorischen Drucksensors (114) erfasst und das exspiratorische Magnetventil (113) steuert, um die Qualitätsstandards der Gasgemischversorgung des Patienten (121) zu gewährleisten.

5. Lungenbeatmungsgerät (100) gemäß den Ansprüchen 1 und 2 **dadurch gekennzeichnet, dass** das Notfall-Atemventil (118) die Luftzufuhr zum Patienten (121) im Fall eines inneren Ausfalls sicherstellt.

6. Lungenbeatmungsgerät (100) gemäß den Ansprüchen 1 und 2 **dadurch gekennzeichnet, dass** das Überdruckventil (119) die Entladung des Gemisches ermöglicht, wenn der Druck den gewünschten Grenzwert überschreitet.

7. Lungenbeatmungsgerät (100) gemäß Anspruch 1, **dadurch gekennzeichnet, dass** das Steuermodul (112) einen Volumenregelungsmodus, einen Druckregelungsmodus, einen druckgeregelten Volumenregelungsmodus und einen Druckunterstützungsmodus beinhaltet.

8. Lungenbeatmungsgerät (100) gemäß Anspruch 1, **dadurch gekennzeichnet, dass** das Steuermodul (112) das Volumen des dem Patienten zugeführten Gasgemisches in einem Bereich von 250 bis 800 ml pro Atemzyklus einstellt.

9. Lungenbeatmungsgerät (100) gemäß Anspruch 1, **dadurch gekennzeichnet, dass** das Steuermodul (112) die Sauerstoffkonzentration im Gasgemisch regelt und eine Versorgung mit Werten zwischen 21% und 100% sicherstellt.

10. Lungenbeatmungsgerät (100) gemäß Anspruch 1, **dadurch gekennzeichnet, dass** das Steuermodul (112) die Anzahl der Atemzüge pro Minute regelt und eine Einstellung zwischen 5 und 50 Zyklen pro Minute ermöglicht.

11. Lungenbeatmungsgerät (100) gemäß Anspruch 1, **dadurch gekennzeichnet, dass** es weiterhin ein Kommunikationsmodul umfasst, um eine Remote-Netzwerkintegration sowie eine Kontrolle und Überwachung von Patienten zu ermöglichen.

## Revendications

1. Ventilateur pulmonaire (100) destiné à être utilisé chez des patients (121), comprenant :
- un module de commande (112) ;
- une entrée d'air comprimé (101) ;
- une entrée O₂ (102) ;
- un capteur de pression d'air (105) et un capteur de pression O₂ (106), lu par le module de commande (112), de manière à assurer la stabilisation du niveau de pression d'entrée d'air comprimé et du niveau de pression d'entrée O2;
- un réservoir O₂ (104), alimenté par l'entrée O₂ (102), le réservoir O₂ (104) étant disposé entre l'entrée O₂ (102) et le capteur de pression d'oxygène (106), approprié pour permettre un stockage O₂ sous pression ;
- un régulateur de pression de filtre (103), alimenté par l'entrée d'air comprimé (101), le régulateur de pression de filtre (103) étant disposé entre l'entrée d'air comprimé (101) et le capteur de pression d'air (105), approprié pour filtrer l'air fourni vers celui-ci,
- une électrovanne d'air (107), alimentée par le régulateur de pression de filtre (103), commandée par le module de commande (112) ;
- un capteur d'écoulement d'air (109), alimenté par l'électrovanne d'air (107) et lu par le module de commande (112) ;
- une électrovanne O₂ (108), alimentée par le réservoir O₂ (104), commandée par le module de commande (112) ;
- un capteur d'écoulement O₂ (110), alimenté par l'électrovanne O₂ (108) et lu par le module de commande (112) ; et
- une conduite de mélange gazeux (111) pour la fourniture d'un mélange gazeux à un patient (121), alimentée par le capteur d'écoulement d'air (109) et par le capteur d'écoulement O₂ (110) qui sont commandés au moyen de données fournies au module de commande (112).

2. Ventilateur pulmonaire (100) selon la revendication 1, **caractérisé en ce qu'**il comprend en outre un capteur de pression du mélange inspiratoire (116), un capteur de concentration d'oxygène (117), une valve respiratoire d'urgence (118) et une valve de surpression (119).

3. Ventilateur pulmonaire (100) selon la revendication 1, **caractérisé en ce qu'**il comprend en outre un capteur d'écoulement expiratoire (115), un capteur de pression expiratoire (114), une électrovanne expiratoire (113) et une sortie de mélange gazeux inspiratoire (120).

4. Ventilateur pulmonaire (100) selon la revendication 1, **caractérisé en ce que** le module de commande (112) lit les données provenant du capteur de pression du mélange inspiratoire (116), du capteur de concentration d'oxygène (117), du capteur d'écoulement expiratoire (115), du capteur de pression expiratoire (114) et commande l'électrovanne expiratoire (113), de manière à assurer les normes de qualité de la fourniture de mélange gazeux au patient (121).

5. Ventilateur pulmonaire (100) selon les revendications 1 et 2 **caractérisé en ce que** la valve respiratoire d'urgence (118) assure la fourniture d'air au patient (121) en cas de panne interne.

6. Ventilateur pulmonaire (100) selon les revendications 1 et 2 **caractérisé en ce que** la valve de surpression (119) permet les rejets du mélange lorsque la pression dépasse la limite souhaitée.

7. Ventilateur pulmonaire (100) selon la revendication 1, **caractérisé en ce que** le module de commande (112) comprend un mode de commande du volume, un mode de commande de la pression, un mode de commande du volume régulé en pression, et un mode d'aide à la pression.

8. Ventilateur pulmonaire (100) selon la revendication 1, **caractérisé en ce que** le module de commande (112) règle le volume du mélange gazeux délivré au patient dans une plage de 250 à 800 mL par cycle respiratoire.

9. Ventilateur pulmonaire (100) selon la revendication 1, **caractérisé en ce que** le module de commande (112) ajuste la concentration en oxygène dans le mélange gazeux assurant une fourniture avec des valeurs comprises entre 21% et 100%.

10. Ventilateur pulmonaire (100) selon la revendication 1, **caractérisé en ce que** le module de commande (112) régule le nombre d'inspirations par minute, permettant le réglage entre 5 et 50 cycles par minute.

11. Ventilateur pulmonaire (100) selon la revendication 1, **caractérisé en ce qu'**il comprend en outre un module de communication pour permettre l'intégration réseau à distance, la commande et la surveillance des patients.
